# EUROPEAN PATENT SPECIFICATION

(11) **EP 0 798 382 B1**
(45) Date of publication and mention of the grant of the patent: **12.11.2003**
(21) Application number: 96302227.2
(22) Date of filing: 29.03.1996
(51) Int. Cl.: C12P 7/06, C12N 1/19, C12N 15/04

(54) **Novel strains of yeast of genus- saccharomyces species- cerevisiae and a process for the preparation of such strains of yeast**
Neue Hefestämme von des Genus Saccharomyces Cerevisiae und Verfahren zur Herstellung derselben
Nouvelles souches de levure du genre Saccharomyces Cerevisiae et procédé pour la préparation de telles souches

(43) Date of publication of application: 01.10.1997
(73) Proprietor: Council of Scientific and Industrial Research, New Delhi 110 001 (IN)
(72) Inventor: Mondal, Alok Kumar, Institute of Microbiological, Chandigargh (IN); Prasad, Grandham Satyanarayana, Institute of, Chandigargh (IN); Chakrabarti, Tapan, Institute of Microbiological, Chandigargh (IN)
(74) Representative: Hallybone, Huw George

(56) References cited:
- FR-A- 2 616 445
- US-A- 4 910 144
- CHEMICAL ABSTRACTS, vol. 99, no. 3, 18 July 1983 Columbus, Ohio, US; abstract no. 20888t, SEKI TATSUJI: "GENETIC CONSTRUCTION OF YEAST STRAINS" page 488; column 2; XP002012310 & BIOTECHNOL. LETT, vol. 5, no. 5, 1983, pages 351-6,

## Description

### FIELD OF THE INVENTION

The invention relates to a new strain of yeast saccharomyces cerevisiae having the accession number MTCC Y0022B211 (= NCYC 2647) and a process for the preparation of such new strain of yeast Saccharomyces cerevisiae useful for the preparation of ethanol by the fermentation of sugars. The ethanol so prepared is useful both for potable and industrial purposes.

### BACKGROUND OF THE INVENTION

It is well known that ethanol or ethyl alcohol (C₂H₅OH) is produced by both fermentation and synthetic methods. Fermentation techniques for ethanol production developed during the early part of this century were supplemented by synthetic processes based on crude petroleum, as oil was much cheaper and abundantly available. However, of late, it is realised that petroleum oil reserve is not going to last long and fermentative production of ethanol has again picked up, using various kinds of renewable fermentable substrates be it (i) sugar (from sugar-cane, sugar beet, fruit) which may be converted to ethanol directly; (ii) starch (from grain, root crops) which are first hydrolysed to fermentable sugars by enzymes; and (iii) cellulose (from wood, agricultural wastes, etc.) which are converted to sugars. (Biotechnology: Economic and Social Aspects :- Issues for Developing Countries. Eds E.J. Da Silva, C..Raticdge and A. Sesson; Cambridge University Press, p24 1992).
Ethanol production by fermentation is based mainly on yeasts, and for large scale fuel production, these are generally of the genus Saccharomyces.
The distillers all over the world would like to have a fermentation process that gives high percentage of alcohol in the broth without sacrificing fermentation efficiency. The primary benefits of such an operation are :
a) potential to increase the production of the fermenter (wherein productivity is defined as amount of alcohol produced per unit fermenter vol./hr).
b) saving in energy/steam requirement during distillation; energy being the major variable cost in distillery operation.
c) reduction in the volume of effluent.
d) in case of flocculent yeast strains, fermentation may be done by recycling of cells, in a continuous mode or by conventional batch mode. Because of their flocculent nature, cells are easily separated from the fermented broth and in a recycling mode or in a continuous mode less sugar is expected to be required for building up of biomass.
In conventional method of ethanol production, initial concentration of sugar in the broth is kept between 14 to 16 percent. Sugar concentrations higher than this is detrimental to growth of yeast strains used in conventional process and fermentation is thus affected. After completion of fermentation, 6.5 to 8 percent alcohol is obtained in the wash. Alcohol is then recovered by distillation using steam.
In order to achieve higher ethanol level in the wash, initial sugar concentration in the broth should also be higher, thereby increasing the osmolarity of the medium which is detrimental to the growth and fermentation of the conventional yeast strains. Conventional strains are also sensitive to high level of ethanol in the broth and they do not have flocculent characteristic.
The main drawback of conventional yeast strains is their failure to grow and remain active at high sugar concentration during fermentation process producing ethanol. At the same time, unless high sugar concentration is used in the fermentation process, it is not possible to obtain increased level of ethanol. Concurrent with this problem, conventional yeasts will not be effective in the fermentation, if ethanol concentration is increased in the broth. The cumulative effects of these problems in the conventional process of production of ethanol is low level of ethanol produced in the wash and consequently, consumption of steam per litre of ethanol distilled is high. Therefore, the processes are not efficient and also not economical. Moreover, separation of conventional yeast strains from fermented broth is not easy and may not be very efficient in a cell-recycling or continuous mode of operation.

### PRIOR ART OF THE INVETION

A strain of Saccharomyces cerevisiae designated as MTCC Y0001 was developed and deposited at the Microbial Type Culture Collection and Gene Bank located at the Institute of Microbial Technology, Chandigarh, India, a constituent laboratory of Council of Scientific and Industrial Research, India. This strain has also been deposited on the 11th March, 1996 at the National Collection of Yeast Culture (NCYC), an International Depository Authority (IDA) under Budapest Treaty and has been assigned the following NCYC No.2646. This strain is osmotolerant, ethanol tolerant and produces high percentage of alcohol and is the subject of our copending Indian patent application number 748/DEL 93.
Another Saccharomyces cerevisiae having accession number MTCC Y0002 (=ATCC 90506) was used for producing the novel and improved varieties of strains of Saccharomyces cerevisiae designated as MTCCY0001. This strain MTCCY0002 has auxotrophic markers, resistance to an antibiotic, flocculating, defective in karyogamy, is haploid and of alpha mating type.
Realising the need to have strains possessing various desirable properties and useful for high density fermentation, we continued our research with the objective of developing flocculent strains of yeast which, in addition to tolerating high initial sugar concentration, i.e. having osmotolerant characteristic, could survive in the higher concentration of ethanol it produces in the broth. In other words, the strain to be used should be tolerant to high concentration of ethanol produced during the fermentation. For use in a versatile mode of operations, flocculent characteristic in the strian is also desirable. In order to run fermentation in cell-recycling, repeated batch fermentation or continuous mode, it is desirable that the strain is of flocculant type in addition to retaining other desirable properties. Envisaged advantage of such a characteristic and unique strain is that less sugar is utilized to build-up the biomass and the sugar thus saved may be converted to ethanol. Moreover, if such a strain is flocculating, there is less wash-out of the cell during operation of the process. Such a strain should also give higher productivity of ethanol. In addition, high concentration of ethanol in the wash results in a reduction in steam requirements for distillation. Since the strains used in conventional distilleries make 6.5 to 8 per cent alcohol in the wash and have a fermentation efficiency of 82.90 - 86.90 per cent as reported by Arbatti, S. V. and Kale, V.M. in the Proceedings of International Seminar on Modernization of Distilleries and Breweries, (organized by All India Distillers Association, July 1969) we concentrated our research for the development of improved strain of Saccharomyces cerevisiae which will have flocculent characteristic and will overcome other problems associated with the use of conventional yeast, and make 7 to 12% or more alcohol in the wash with a fermentation efficiency which is as good as, if not better than, the conventional process.

### SUMMARY AND OBJECTS OF THE INVENTION

The main object of the present invention is, therefore, to provide a novel strain of genus - Saccharomyces species- cerevisiae designated as MTCC Y0022B211 (=NCYC 2647) which is produced by genetic hybridisation and cytoduction and is useful in the fermentation process for the preparation of ethanol.

Another object of the present invention relates to a process for the preparation of a novel strain of genus - Saccharomyces species- cerevisiae designated as MTCC Y0022B211 (=NCYC 2647) which is produced by genetic hybridisation and cytoduction and is useful in the fermentation process for the preparation of ethanol.

The new strain has been deposited in the National Facility on Microbial Type Culture Collection and Gene Bank (MTCC) located at the Institute of Microbial Technology, Chandigarh, India, a constituent laboratory of Council of Scientific and Industrial Research, India. This strain has also been deposited at National Collection of Yeast Cultures (NCYC), U.K. on 11th March 1996, an IDA under Budapest Treaty and has been assigned the number NCYC 2647.

### DETAILED DESCRIPTION OF THE INVENTION

In general, desirable genetic properties of two strains MTCC Y0001 (=NCYC 2646) and MTCC Y0002 (=ATCC 90506) or more may be brought together, for creating novel strains by many ways such as, mating, crossing, genetic hybridisation, cytoduction, protoplast fusion as well as by genetic engineering and expression of the cloned gene(s). In doing this manipulations in the laboratory, the strain needs to be properly marked and an innovative method has to be developed for the production of improved and novel strain.
The starting yeast strains of the present process i.e. MTCC Y0001 and MTCC Y0002 as well as the new strains have been deposited in the National Facility on Microbial Type Culture Collection and Gene Bank (MTCC) located at the Institute of Microbial Technology, Chandigarh, India, a constituent laboratory of Council of Scientific and Industrial Research, India. The yeast strain MTCC Y0001 was also deposited on 11th March 1996 and the National Collection of Yeast Culture, U.K. and has been awarded the accession number NCYC 2646.
Accordingly, the present invention provides a process for the preparation of a novel strain of yeast Saccharomyces cerevisiae having the accession number MTCC Y0022B211 (=NCYC 2647) deposited at the Microbial Type Culture Collection and Gene Bank located at the Institute of Microbial Technology, Chandigarh, India, one of the constituent laboratory of Council of Scientific and industrial Research, India, and also at the National Collection of Yeast Culture, U.K, an International Depository Authority under Budapest Treaty, which process comprises,
a) growing a diploid strain of yeast Saccharomyces cerevisiae designated as MTCC Y0001 (=NCYC 2646) in a known medium, sporulating the strain by conventional method, treating the sporulated cells with a known lytic enzyme, collecting the liberated spores,
b) growing a haploid strain of yeast Saccharomyces cerevisiae designated as MTCC Y0002 (=ATTC 90506) by known method, collecting the cells in a conventional medium,
c) mixing the spores obtained in step (a) with the cells obtained in step (b), incubating the resultant spore-cell mixture at a temperature in the range of 15 to 37°C for a period of 1 to 10 days,
d) spreading the incubated spore-cell mixture over a known non-selective medium, and incubating the said mixture at a temperature in the range of 15 to 37°C for a period of to 10 days,
e) collecting the cells produced in step(d), and spreading over a selective medium so as to eliminate the spores/cells of steps (a) and (b) and allowing only hybrid cells/cytoductants to grow, and
f) purifying the hybrid cells/cytoductants by conventional methods.

The medium used for sporulating the diploid strain MTCC Y0001 may consist of agar, yeast extract, dextrose, potassium acetate, and distilled water and the lytic enzyme used may be selected from lyticase, glusulase or zymolyase.

The haploid strain MTCC Y0002 used in step(b) may be selected from a haploid strain as such or haploid obtained from diploid strains. By way of example, other properly marked strains may be used.

The haploid strain in step (b) is grown in a known medium such as YEPD ( Yeast extract, peptone, dextrose) at a temperature in the range of 15 to 35°C for a period of 1 to 10 days. The ratio of the strains MTCC Y0001 and MTCC Y0002 used may range from 20:1 to 1:20.

The incubation in steps (c) and (d) may be effected at a temperature in the range of 15 to 37°C for a period in the range of 1 to 10 days. The non-selective medium used in step (d) may be selected from SD Medium (yeast nitrogen base, dextrose, agar and distilled water), YPD (yeast extract, peptone, dextrose, agar and distilled water) or YPG (yeast extract, peptone, glycerol, agar and distilled water).

The selective medium used in step(e) is a conventional medium such as SDG (yeast nitrogen base, dextrose, glycerol, agar and distilled water) fortified with broad range antibiotic such as geniticin, oligomycin, chloramphenicol and the likes.

The different strains are separated by conventional methods like streaking or dilution plating.

The details of the steps of the proces of the present invention are as follows.

The parental strain designated as MTCC Y0001 is homothallic and produces spores of both a mating, type and alpha mating type. This strain was sporulated an appropriate media and the spores were released by dissolving the ascus wall by enzymatic treatment and spores were collected. These spores and the cells (spores) of the other strain designated as MTCG Y0002 are mixed in different ratios. After proper incubation at a temperature in the range of 10 to 37°C, the mixture is spread over non-selective medium and then the population of cells were spread on selective medium. The selective medium was designed such that the original two strains (Y0001 and Y0002) would not survive. Hybrids only created by transfer of desirable genetic material from Y0001 to Y0002 and vice versa would be able to grow and there were many such hybrids or cytoductants. The hybrids or cytoductants produced were then initially checked for flocculation. Some of them showed flocculation while others did not. Among the flocculent hybrids(or cytoductants), three types were apparent. These are (i) good, sedimentation time 10-20 seconds (ii) moderate, sedimentation time 20-35 seconds and (iii) slow, sedimentation time 35-60 seconds.

The strain is novel and its characteristics are given in Table 1.

**Table 1**

| | Hybrid/cytoductant |
|---|---|
| Antibiotic resistance | + |
| Auxotrophy | - |
| Flocculation | + |

| Growth on | |
|---|---|
| a) 40% molasses | + |
| b) 50% molasses | + |
| Ethanol production | 7-12% |

Electrophoretic karyotyping of the hybrid appears to be different from MTCC Y0001 and MTCC Y0002.
It has been assigned the following accession number
MTCC Y0022B211 (= NCYC2647)

The new strain prepared by the process of the present invention has the following characteristics:
(i) it grows at a temperature ranging between 15 degree C to 37 degree C in Yeast Extract Peptone Dextrose (YEPD) medium with 2% glucose,
(ii) it grows on agar plates containing molasses concentration of upto 50% and does not need any supplementation when grown in this medium,
(iii) it produces 7% to 12% v/v ethanol at 30 degree C with 90% efficiency or more,
(iv) it also grows in Yeast Extract Peptone Dextrose (YEPD) medium in presence of 12% ethanol. The strain is, therefore, osmotolerant, ethanol, tolerant and produces high level of alcohol,
   In liquid medium, like Yeast Extract Peptone Dextrose or Yeast Extract Peptone Sucrose (YEPS) or molasses the cells show flocculation,
vi) it is resistant to an antibiotic,
vii) it sporulates and produces ascospores,
viii) properties described above are quite stable,
ix) it retains its fermentation ability for use in recycling,
x) under appropriate conditions of flocculation, it settles or sediments within 10 seconds to 55 seconds.
The biochemical properties of the new strain are that it utilizes glucose, sucrose, maltose, saccharose and raffinose as carbon sources but does riot grow on salicin, lactose, inositol, citrate, 2-Keto-D-gluconate, arabinose, xylose, adanitol, xylitol, sorbitol, methyl-D-glycoside, n-acetyl-glucosamine, cellobiose, trehalose and melizitose. Growth was poor when sodium nitrate, potassium nitrate or lysin was used as a sole source of nitrogen.
Molasses is a by product of sugar manufacturing process. What remain after sugar is extracted from sugar cane juice is known as molasses. This byproduct still contain some sugar, concentration of which depends on the efficiency of sugar extraction. A good quality (Grade A) molasses contain a minimum of 55% (w/v) sugar. A Grade C molasses on the other hand, has about 40% (w/v) sugar. In order to achieve a desired sugar concentration i a fermenter, molasses is accordingly diluted with water.

Since ethanolic fermentation is a process by which certain microorganisms convert sugars such as sucrose, glucose and fructose to ethyl alcohol, the sugars used in the process of the present invention can be glucose, sucrose, fructose or other reducing sugars as such or as present in molasses or a combination of these or the sugars obtained from starch and other lignocellulosic material.
It could be observed from the description given above that the process of the present invention results in a new strain which has improved characteristics which can be utilised for the production of high percentage of ethanol. The process results in products having synergistic characteristics.
The process of the present invention is illustrated in the examples given below which should not however be construed to limit the scope of the present invention.

### Example 1:

A strain of Saccharomyces cerevisiae MTCC Y0001 (=NCYC 2646) was grown in YEPD (yeast extract, peptone, dextrose, distilled water) medium and then spread on presporulation medium (yeast extract, peptone, dextrose, agar, distilled water) and incubated for 48 hours at 25°C. Cells were harvested and were then spread over sporulation medium and incubated at 25°C and samples were observed under microscope to sec the progress of sporulation which usually takes 3 to 7 days. The asci (structure containing spores) are then harvested, treated with zymolyase enzyme and a random spore suspension is made. The other strain MTCC Y0002 (=ATCC 90506) is grown ill YEPD. These two suspensions ar mixed in 1:10 ratio, spread on YEPD medium and incubated at 25°C for 16 hours. The mixture is then spread on Complete Yeast Nitrogen Base Glucose medium and incubated at 25°C. Cells from above plates are harvested and then spreaded on Yeast Nitrogen Base Glycerol (YNBG) medium containing oligomycin. Cells growing on these plates are considered hybrids or cytoductants and they are then checked for transfer of desired genetic properties.
Hybrids or cytoductants as generated by methods described above are purified to single colonies on YNBG selective plates.
Cells are then inoculated in 2 ml YEPD medium in 24-well micro litre plates. The plates are incubated at 30°C with shaking at 150 rpm for 24 to 48 hrs. Flocculation is observed as accumulation of majority of the cells as small granules at the bottom of the wells.
This flocculation can also be visualised in flasks containing liquid medium like YEPD, YEPS, buffered YNB Glucose, molasses etc.
Genetically improved hybrid (cytoductants) was grown in YEPD for 16 hrs at 30°C. Cells from this culture were inoculated separately into 100 ml of clarified molasses in conical flasks, incubated at 30°C with shaking at 150 rpm. Growth and fermentation, as determined by the loss of weight of the flasks, was monitored at regular intervals upto 24 hrs. The contents of each flask was quickly poured into graduated measuring cylinders. Time taken for the flocks to sediment at the bottom was recorded with the help of a stop watch. For example, MTCC Y0022B211 took 34 to 35 seconds.
The new strain is inoculated in YEPD and incubated at 25° C with shaking for 24 hrs. Samples from each are added to fresh YEPD medium containing 17 percent sugar. The flasks are incubated at 30°C with shaking. Samples are withdrawn at intervals and incubation continued upto 48 hrs. Sugar contents and alcohol produced in each sample is determined by standard anthrone method and potassium dichromate methods respectively. This strain showed flocculant property. It is observed that the present strain produces alcohol between 7 percent to about 12 percent.

### Example 2

A strain of Saccharomyces cerevisiae MTCC Y0001 (=NCYC 2646) was grown in liquid YEPD medium for 16 hours at 30°C. The cells were then transferred in a flask containing liquid presporulation medium and incubated at 30°C for 16 hours. Then the cells were transferred to liquid sporulation medium in a flask and were incubated at 30°C for 2 to 5 days. When majority of the cells were found to form asci, each containing 2 to 4 spores, the cells (asci) were centrifuged, suspended in a buffer and treated with lyticase enzyme. The spores thus released were harvested by centrifugation and then made into a suspension. The other strain MTCC Y0002 (=ATCC 90506) is grown in YEPD. These two suspensions are mixed in 10:1 ratio, spread on YEPD plates and incubated at 30°C for 16 hours. The mixture is then spread on Complete Yeast Nitrogen Base Glucose (YNBG) medium and incubated at 30°C. Cells from above plates are harvested and then spreaded on selective plates (YNB Glycerol containing oligomycin). Cells growing on these plates are considered hybrids or cytoductants and they are then checked for transfer of desired genetic properties.
Hybrids or cytoductants as generated by methods described above are purified to single colonies on selective plates. Cells are then inoculated in 2 ml YEPD medium in 24-well microtitre plates. The plates are incubated at 30°C with shaking at 150 rpm for 24 to 48 hrs. Flocculation is observed as accumulation of majority of the cells as small granules at the bottom of the wells.
This flocculation can also be visualised in flasks containing liquid medium like YEPD, YEPS, buffered YNBG, molasses etc.
Genetically improved hybrid (cytoductant) was grown in YEPD for 16 hrs at 30°C. Cells from this culture were inoculated separately into 100 ml of clarified molasses in conical flasks, incubated at 30°C with shaking at 150 rpm. Growth and fermentation, as determined by the loss of weight of the flasks, was monitored at regular intervals upto 24 hrs. The contents of each flask was quickly poured into graduated measuring cylinders. Time taken for the flocks to sediment at the bottom was recorded with the help of a stop watch and found to be good.
The new strain is inoculated in YEPD and incubated at 30°C with shaking for 15 to 24 hrs. Samples from each are added to fresh YEPD medium containing 17 percent sugar. The flasks are incubated at 30°C with shaking. Samples are withdrawn at intervals and incubation continued upto 48 hrs. Sugar contents and alcohol produced in each sample is determined by standard anthrone method and potassium dichromate methods respectively. This strain showed flocculent property. It is observed that the present novel strain produces alcohol between about 7 percent to about 12 percent.

### Example 3

A strain of Saccharomyces cerevisiae MTCC Y0001 (=NCYC 2646) was grown in YM (yeast extract, peptone, malt extract, dextrose, distilled water) medium. Cells were harvested and were then spread over sporulation medium and incubated at 32°C and samples were observed under microscope to see the progress of sporulation which usually takes 4 to 10 days. The asci (structure containing spores) are then harvested, treated with zymolyase enzyme and a random spore suspension is made. The other strain MTCC Y0002 (=ATCC 90506) is grown in YEPD. These two suspensions are mixed in 1:1 ratio and centrifuged. The pellet was then kept at room temperature (25°C) for two hours and was inoculated in YEPD medium. Cells from above culture are harvested and then spraded on selective plates (YNB Glycerol containing oligomycin). Cells growing on these plates are considered hybrids or cytoductants and they are then checked for transfer of desired genetic properties.

Hybrids or cytoductants as generated by methods described above are purified to single colonies on selective plates. Cells are then inoculated in 2 ml YEPD medium in 24-well microtitre plates. The plates are incubated at 30°C with shaking at 150 rpm for 24 to 48 hrs. Flocculation is observed as accumulation of majority of the cells as small granules at the bottom of the wells.
This flocculation can also be visualised in flasks containing liqiud medium like YEPD, YEPS, buffered YNBG, molasses etc.
Genetically improved hybrid (cytoductant) was grown in YEPD for 16 hours at 30°C. Cells from these cultures were inoculated separately into 100 ml of clarified molasses in conical flasks, incubated at 30°C with shaking at 150 rpm. Growth and fermentation, as determined by the loss of weight of the flasks, was monitored at regular intervals upto 24 hrs. The contents of each flask were quickly poured into graduated measuring cylinders. Time taken for the flocks to sediment at the bottom was recorderded with the help of a stop watch and found to be good.
The new strain is inoculated in YEPD and incubated at 30°C with shaking for 15 to 24 hrs. Samples from each are added to fresh YEPD medium containing 17 percent sugar. The flasks are incubated at 30°C with shaking. Samples are withdrawn at intervals and incubation continued upto 48 hours. Sugar contents and alcohol produced in each sample is determined by standard anthrone method and Potassium dichromate methods respectively. This strain showed flocculent property. It is observed that the present novel strain produces alcohol between about 7 percent to about 12 percent.

### Advantages of the Invention:

1. The new strain of yeast produced by the process has flocculent property and as a result, majority of the cell sediment at the bottom when agitation is stopped.
2. Since cells are already grown-up in the process, less sugar is utilised for further growth and this may lead to availability into alcohol in a continuous or recycling on repeated batch fermentation mode.
3. In addition, the new yeast strain produced is more osmotolerant, more ethanol tolerant and as a result, produces higher level of alcohol compared to conventional yeast strains without any loss of their ability for conversion of sugar to alcohol.
4. Because of above said properties of the new strain produced, higher initial sugar concentration can be used for fermentation. Consequently for a given capacity plant, alcohol output may be higher depending on the conditions used.
5. Because of high alcohol content in the wash, which is achieved by high gravity fermentation, there is substantial reduction in steam consumption in the recovery process, net saving of steam in distillation process can be between 0.8 kg to 1.2 kg per litre of alcohol distilled.
6. Consequent to high gravity fermentation, there is a net reduction in effluent volume. This may result in a more compact effluent treatment plant.
7. The new strain is genetically marked and because of this, the strain is easily identifiable.

## Claims

1. A strain of yeast Saccharomyces cerevisiae having the accession number MTCC Y0022B211 (=NCYC 2647) which is useful for the preparation of ethanol by the fermentation of sugars.

2. A process for the preparation of a strain of yeast Saccharomyces cerevisiae having the accession number MTCC Y0022B211 (=NCYC 2647) which comprises,
a) growing a diploid strain of yeast Saccharomyces cerevisiae designated as MTCC Y0001 (=NCYC 2646) in a medium, sporulating the strain by conventional method, treating the sporulated cells with a lytic enzyme, collecting the liberated spores,
b) growing a haploid strain of yeast Saccharomyces cerevisiae designated as MTCC Y0002 (=ATTC 90506) and collecting the cells in a conventional medium,
c) mixing the spores obtained in step (a) with the cells obtained in step (b), incubating the resultant spore-cell mixture at a temperature in the range of 15 to 37°C for a period of 1 to 10 days,
d) spreading the incubated spore-cell mixture over a non-selective medium, and incubating the said mixture at a temperature in the range of 15 to 37°C for a period of 1 to 10 days,
e) collecting the cells produced in step (d), and spreading over a selective medium so as to eliminate the spores/cells of steps (a) and (b) and allowing only hybrid cells/cytoductants to grow, and
f) purifying the hybrid cells/cytoductants by a conventional method.

3. A process as claimed in claim 2 wherein the haploid strain MTCC Y0002 used in step (b) is selected from a haploid strain as such or haploid obtained from diploid strains.

4. A process as claimed in claim 2 wherein the haploid strain in step (b) is grown in a known medium such as YEPD (Yeast extract, peptone, dextrose) at a temperature in the range of 15 to 35°C for a period of 1 to 10 days.

5. A process as claimed in claim 2 wherein the incubation in steps (c) and (d) is effected at a temperature in the range of 15 to 37°C for a period in the range of 1 to 10 days.

6. A process as claimed in claim 2 wherein the selective medium used in step (e) is a conventional medium such as SDG (yeast nitrogen base, dextrose, glycerol, agar and distilled water) fortified with broad range antibiotic such as geniticin, oligomycin or chloramphenicol.

## Patentansprüche

1. Hefestamm *Saccharomyces cerevisiae* mit der Zulassungsnummer MTCC Y0022B211 (=NCYC 2647), welcher zur Herstellung von Ethanol durch die Fermentation von Zuckern geeignet ist.

2. Verfahren zur Herstellung eines *Saccharomyces cerevisiae* Hefestammes mit der Zulassungsnummer MTCC Y0022B211 (=NCYC 2647), umfassend
(a) das Wachsen eines diploiden, als MTCC Y0001 (=NCYC 2646) bezeichneten *Saccharomyces cerevisiae* Hefestammes in einem Medium, das Bilden von Sporen des Stammes durch ein herkömmliches Verfahren, das Behandeln der Sporen gebildet habenden Zellen mit einem lytischen Enzym, das Sammeln der freigesetzten Sporen,
(b) das Wachsen eines haploiden, als MTCC Y0002 (=ATTC 90506) bezeichneten *Saccharomyces cerevisiae* Hefestammes und das Sammeln der Zellen in einem herkömmlichen Medium,
(c) das Mischen der in Schritt (a) erhaltenen Sporen mit den in Schritt (b) erhaltenen Zellen, das Inkubieren des resultierenden Sporen/Zellgemisches bei einer Temperatur im Bereich von 15 bis 37°C für eine Zeitdauer von 1 bis 10 Tagen,
(d) das Verteilen des inkubierten Sporen-Zellgemisches auf einem nichtselektiven Medium und das Inkubieren des Gemisches bei einer Temperatur im Bereich von 15 bis 37°C für eine Zeitdauer von 1 bis 10 Tagen,
(e) das Sammeln der in Schritt (d) hergestellten Zellen und das Verteilen auf einem selektiven Medium, um die Sporen/Zellen von Schritt (a) und (b) zu eliminieren, und das Zulassen, daß nur Hybridzellen/Cytoducten wachsen, und
(f) das Reinigen der Hybridzellen/Cytoducten durch ein herkömmliches Verfahren.

3. Verfahren nach Anspruch 2, wobei der in Schritt (b) verwendete haploide Stamm MTCC Y0002 aus einem an sich haploiden Stamm oder einem durch diploide Stämme erhaltenen haploiden Stamm ausgewählt ist.

4. Verfahren nach Anspruch 2, wobei der haploide Stamm in Schritt (b) in einem bekannten Medium wie YEPD (Hefeextrakt, Pepton, Dextrose) bei einer Temperatur im Bereich von 15 bis 35°C für eine Zeitdauer von 1 bis 10 Tagen wächst.

5. Verfahren nach Anspruch 2, wobei die Inkubation in den Schritten (c) und (d) bei einer Temperatur im Bereich von 15 bis 37°C für eine Zeitdauer im Bereich von 1 bis 10 Tagen ausgeführt wird.

6. Verfahren nach Anspruch 2, wobei das in Schritt (e) verwendete selektive Medium ein herkömmliches Medium wie SDG (Hefenitrogenbase, Dextrose, Glycerol, Agar und destilliertes Wasser), angereichert mit einem Breitbandantibiotikum wie Geniticin, Oligomycin oder Chloramphenicol, ist.

## Revendications

1. Souche de levure *Saccharomyces cerevisiae* ayant le numéro d'accès MTCC Y0022B211 (=NCYC 2647) qui est utile pour la préparation d'éthanol par fermentation de sucres.

2. Procédé de préparation d'une souche de levure *Saccharomyces cerevisiae* ayant le numéro d'accès MTCC Y0022B211 (=NCYC 2647), qui comprend les étapes consistant à
a) cultiver une souche diploïde de levure *Saccharomyces cerevisiae* désignée par MTCC Y0001 (=NCYC 2646) dans un milieu, sporuler la souche par un procédé classique, traiter les cellules sporulées avec une enzyme lytique, recueillir les spores libérés,
b) cultiver une souche haploïde de levure *Saccharomyces cerevisiae* désignée par MTCC Y0022 (=ATTC 90506) et recueillir les cellules dans un milieu classique,
c) mélanger les spores obtenus dans l'étape (a) avec les cellules obtenues dans l'étape (b), incuber le mélange de spores et de, cellules résultant à une température dans la gamme de 15 à 37°C pendant une durée de 1 à 10 jours,
d) étaler le mélange de spores et de cellules incubé sur un milieu non sélectif et incuber ledit mélange à une température dans la gamme de 15 à 37°C pendant une durée de 1 à 10 jours,
e) recueillir les cellules produites dans l'étape (d) et les étaler sur un milieu sélectif de manière à éliminer les spores/cellules des étapes (a) et (b) et à ne laisser proliférer que les cellules hybrides/cytoductants, et
f) purifier les cellules hybrides/cytoductants par un procédé classique.

3. Procédé selon la revendication 2 dans lequel la souche haploïde MTCC Y0002 utilisée dans l'étape (b) est choisie parmi une souche haploïde telle quelle ou un haploïde obtenu à partir de souches diploïdes.

4. Procédé selon la revendication 2 dans lequel la souche haploïde dans l'étape (b) est cultivée dans un milieu connu tel que YEPD (extrait de levure, peptone, dextrose) à une température dans la gamme de 15 à 37°C pendant une durée de 1 à 10 jours.

5. Procédé selon la revendication 2 dans lequel l'incubation dans les étapes (c) et (d) est effectuée à une température dans la gamme de 15 à 37°C pendant une durée dans la gamme de 1 à 10 jours.

6. Procédé selon la revendication 2 dans lequel le milieu sélectif utilisé dans l'étape (e) est un milieu classique tel que SDG (base azotée de levure, dextrose, glycérol, gélose et eau distillée) fortifié avec un antibiotique à large spectre tel que la géniticine, l'oligomycine ou le chloramphénicol.
